# EUROPEAN PATENT SPECIFICATION

(11) **EP 0 549 702 B1**
(45) Date of publication and mention of the grant of the patent: **15.03.2000**
(21) Application number: 91917632.1
(22) Date of filing: 23.08.1991
(51) Int. Cl.: A61K 38/19

(54) **USE OF RECOMBINANT COLONY STIMULATING FACTOR-1**
VERWENDUNG DES REKOMBINANTEN KOLONIESTIMULIERENDEN FAKTORS-1
UTILISATION DU FACTEUR STIMULATEUR DE COLONIES DE TYPE 1 RECOMBINE

(30) Priority: 23.08.1990 US 572149
(43) Date of publication of application: 07.07.1993
(60) Divisional application: 99201175.9
(73) Proprietor: CHIRON CORPORATION, Emeryville, California 94608 (US)
(72) Inventor: RALPH, Peter, Orinda, CA 94563 (US); CHONG, Kong, T., Ft. Lauderdale 33332 Florida (US); DEVLIN, James, Lafayette, CA 94549 (US); ZIMMERMAN, Robert, Orinda, CA 94563 (US); AUKERMAN, Sharon, Lea, Oakland, CA 94620 (US); RING, David, B., Redwood City, CA 94061 (US); MA, Sylvia, Hsieh, Fremont, CA 94555 (US)
(74) Representative: Bizley, Richard Edward
(86) International application number: US9106052
(87) International publication number: WO9203151

(56) References cited:
- WO-A-89/02746
- The Journal of Immunology, volume 143, no. 2, 15 July 1989 (Baltimore, MD, US) M. Wang et al.: "Enhancement of human monocyte function against Candida albicans by the colony-stimulating factors (CSF): IL-3, granulocyte-macrophage-CSF and macrophage-CSF", pages 671-677, see pages 671-677
- The Journal of Immunology, volume 139, no. 2, 15 July 1987 (Baltimore, MD, US) A. Karbassi et al.: "Enhanced killing of Candida albicans by murine macrophages treated with macrophage colony-stimulating factor: evidence for augmented expression of mannose receptors", pages 417-421, see pages 417-421
- Biological Abstracts; volume 87, no. 11, 1989 (Philadelphia, PA, US) S. Oka et al.: "Clinical evaluation of Fluconazole", see page AB-205, abstract no. 112788, & Jpn. J.Antibiot. 42(1):31-3,1989
- Infection and Immunity, volume 59, no. 3, March 1991 (Washington, DC, US) E. Cenci et al.: "Macrophage colony stimulating factor in murine candidiasis: serum and tissue levels during infecton and protective effect of exogenous administration", pages 868-872, see pages 868-872

## Description

The present invention relates to the therapeutic use of recombinantly produced human colony stimulating factor-1 (CSF-1) being anti-fungal treatments.

The ability of certain factors produced in very low concentration in a variety of tissues to stimulate the growth and development of bone marrow progenitor cells into macrophages and/or granulocytes has been known for nearly 15 years. The presence of such factors in sera, urine samples, and tissue extracts from a number of species is demonstrable using an in vitro assay which measures the stimulation of colony formation by bone marrow cells plated in semi-solid culture medium. There are no acceptable in vivo assays. Because these factors induce the formation of such colonies, the factors collectively have been called Colony Stimulating Factors (CSF).

More recently, it has been shown that there are at least four subclasses of human CSF proteins which can be defined according to the types of cells found in the resultant colonies. One subclass, CSF-1, results in colonies containing predominantly macrophages. Other subclasses produce colonies which contain both neutrophilic granulocytes and macrophages (GM-CSF); which contain predominantly neutrophilic granulocytes (G-CSF); and which contain neutrophilic and cosinophilic granulocytes, macrophages, and other myeloid cell types (basophils, erythrocytes, and megokaryocytes) (IL-3).

GM-CSF is described by Gough, et al., Nature (1984) 309: 763-767. This protein is further described in WO 87/02060, published April 9, 1987, as being useful to treat cancer patients to regenerate leukocytes after traditional cancer treatment, and to reduce the likelihood of viral; bacterial, fungal and parasitic infection in immunocompromised individuals, such as those having acquired immune deficiency syndrome (AIDS). Human IL-3 has been cloned by Yank, Y.C., et al., Cell (1986) 47:3.

There are murine factors analogous to the above human CSFs, including a murine factor called IL-3 which induces colonies from murine bone marrow cells which contain all these cell types plus megakaryocytes, erythrocytes, and mast cells, in various combinations. Murine IL-3 has been cloned by Fung, M.C., et al., Nature (1984) 307:233. See also Yakota. T., et al., Proc Natl Acad Sci (USA) (1984) 81:1070-1074; Wong, G.G.,et al., Science (1985) 228:810-815; Lee, F., et al., Proc Natl Acad Sci (USA) (1985) 82:4360-4364; and Cantrell, M.A., et al., Proc Natl Acad Sci (USA) (1985) 82:6250-6254.) These CSFs and others have been reviewed by Dexter, T.M., Nature (1984) 309:746, and Vadas, M.A., et al., J. Immunol. (1983) 130:793, Clark, S.C., Science (1987) 236:1229, and Sachs, L., Science (1987) 238:1374.

The cloning and expression of G-CSF is described in US Patent No 4,810,643 and a method to purify G-CSF from human oral cancer tissue is described in US Patent No 4,833,127.

Treatment of patients suffering from AIDS with CSF-1, alone or together with erythropoietin and/or an antiviral agent and/or IL-2 is reported in WO87/03204, published June 4, 1987. US Patent No 4,482,485, issued November 13, 1984, states that CSF isolated from human urine can be used for a supporting role in the treatment of cancer. In addition, EP 118,915, published September 19, 1984, reports production of CSF for preventing and treating granulocytopenia and macrophagocytopenia in patients receiving cancer therapy, for preventing infections, and for treating patients with implanted bone marrow.

In addition, CSF-1 is reported to stimulate nonspecific tumoricidal activity (Ralph et al., Immunobiol (1986) 172:194-204). Ralph et al., Cell Immunol (1983) 76:10-21 reported that CSF-1 has no immediate direct role in activation of macrophages for tumoricidal and microbiocidal activities against fibrosarcoma 1023, lymphoma 18-8 and L Tropica amastigotes. Ralph et al., Cell Immunol (1987) 105:270-279 reports the delayed tumoricidal effect of CSF-1 alone and the added tumoricidal effect of a combination of CSF-1 and lymphokine on murine sarcoma TU5 targets. Copending, commonly owned US Application Serial No 126,221, filed February 19, 1988, discloses the synergistic effect of CSF-1 and G-CSF to stimulate the immune system.

In addition, Warren et al., J of Immunol (1986) 137:2281-2285 discloses that CSF-1 stimulates monocyte production of interferon, TNF and colony stimulating activity. Lee et al., J of Immunol (1987) 138:3019-3022 discloses CSF-1 induced resistance to viral infection in murine macrophages.

Wang et al., J Immunol (1989) 143:671-677 reports on *in vitro* experiments wherein human GM-CSF, IL-3 and M-CSF are tested alone in human monocyte cultures for Candida killing activity.

A significant difficulty in putting CSF proteins in general, and CSF-1 in particular, to any useful function has been their unavailability in distinct and characterizable form in sufficient amounts to make their employment in therapeutic use practical or even possible.

The present invention provides the use of synergistically effective amounts of human colony stimulating factor 1 (CSF-1) and an anti-fungal agent, selected from amphotericin B, fluconazole, ketoconazole, miconazole or intraconazole for the manufacture of a medicament for the treatment of fungal infection.

Preferred individuals for the said treatment are immunosuppressed individuals, wherein the immunosuppression of the individuals is due to infection including AIDS, to chemical agents including agents given for cancer chemotherapy or bone marrow transplant, to burn trauma, to other major trauma, or to irradiation.

The fungal infection may be caused by at least one fungus selected from *Candida, Aspergillus, Cryptococcus, Histoplasma, Coccidioides, Paracoccidioides, Mucor, Rhodotorula, Sporothrix or Blastomyces* species.

In particular preferred embodiments the CSF-1 is administrable at a daily dose in the range 0.01 to 50 mg/M², preferably 0.05 to 10 mg/M², more preferably 0.5 to 5 mg/M², even more preferably 0.05 to 2.0 mg/M². In further particularly preferred embodiments the CSF-1 is administrable at a daily dose in the range 0.05 to 2.0 mg/M².

The CSF-1 is preferably administrable for at least 14 days.

The invention herein makes use of recombinant production of CSF-1. This subclass of CSF has been characterized and delineated by specific radioimmunoassays and radioreceptor assays to suppress specifically CSF-1 activity, without affecting the biological activities of the other subclasses, and macrophage cell line J774 contains receptors which bind CSF-1 specifically. A description of these assays was published by Das, S.K., et al., Blood (1981) 58:630.

"Colony stimulating factor-1 (CSF-1 or M-CSF)" refers to those proteins which exhibit the spectrum of activity understood in the art for CSF-1, ie, when applied to the standard in vitro colony stimulating assay of Metcalf, D., J. Cell Physiol. (1970) 76:89, it is capable of stimulating the formation of primarily macrophage colonies. Native CSF-1 is a glycosylated dimer; dimerisation is reported to be necessary for activity as the monomer is not active in the Metcalf colony stimulating assay (supra) or various other in vitro bioactivity assays (Ralph, P. et al., Blood (1986) 68:633; Stanley, E R., et al; J Biol Chem (1977) 252:4305). As used herein the number of CSF-1 units corresponds to the number of colonies in the mouse bone marrow colony assay in the titratable range (described in Ralph et al., Blood, supra). Contemplated within the scope of the invention and within the definition of CSF-1 are both the dimeric and monomeric forms. The monomeric form may be converted to the dimeric form by in vitro provision of suitable refolding conditions as described in copending PCT WO 88/08003, published October 20, 1988, and in U.S. Patent No. 4,929,700, issued May 29, 1990, and the monomer is per se useful as an antigen to produce anti-CSF-1 antibodies.

There appears to be some species specificity: Human CSF-1 is operative both on human and on murine bone marrow cells; murine CSF-1 does not show activity with human cells. Therefore, "human" CSF-1 should be positive in the specific murine radioreceptor assay of Das, 1981, supra, although there is nor necessarily a complete correlation. The biological activity of the protein will generally also be inhibited by neutralizing antiserum to human urinary CSF-1 (Das, 1981, supra). However, in certain special circumstances (such as, for example, where a particular antibody preparation may recognize a CSF-1 epitope not essential for biological function, and which epitope is not present in the particular CSF-1 mutein being tested) this criterion may not be met.

Certain other properties of CSF-1 have been recognized more recently, including the ability of this protein to stimulate the secretion of series E prostaglandins, interleukin-1, and interferon from mature macrophages (Moore, R., et al., Science (1984) 223:178). The mechanism for these latter activities is nor presently understood, and for purposes of definition herein, the criterion for fulfillment of the definition resides in the ability to stimulate the formation of monocyte/macrophage colonies using bone marrow cells from the appropriate species as starting materials, and under most circumstances (see above), show inhibition of this activity by neutralizing antiserum against purified human urinary CSF-1, and, where appropriate for species type, exhibit a positive response in the radioreceptor assay. (It is known that the proliferative effect of CSF-1 is restricted to cells of mononuclear phagocytic lineage (Stanley, ER., The Lymphokines (1981), Stewart, W.E., II, et al., ed, Humana Press, Clifton, NJ), pp. 102-132) and that receptors for CSF-1 are found on cells of this lineage (Byrne, P.V., et al., Cell Biol (1981) 91:848), placental trophoblasts and some other cells).

"Effective amount" signifies an amount effective to perform the function specified.

"Therapeutic treatment" indicates treating a subject after a disease is contracted, and includes prophylactic therapy.

"Mammals" indicates any mammalian species, and includes rabbits, mice, dogs, cats, primates and humans, preferably humans.

"Immunosuppression" means prevention or diminution of the immune response due to infections, chemical agents, burns, major trauma, or irradiation. Individuals who are immunocompromised are also immunosuppressed.

"Expression system" refers to DNA sequences containing a desired coding sequence and control sequences in operable linkage, so that hosts transformed with these sequences are capable of producing the encoded proteins. In order to effect transformation, the expression system may be included on a vector; however, the relevant DNA may then also be integrated into the host chromosome.

As used herein "cell", "cell line", and "cell culture" are used interchangeably and all such designations include progeny. Thus "transformants" or "transformed cells" includes the primary subject cell and cultures derived therefrom without regard for the number of transfers. It is also understood that all progeny may not be precisely identical in DNA content, due to deliberate or inadvertent mutations. Mutant progeny which have the same functionality as screened for in the originally transformed cell are included. Where distinct designations are intended, it will be clear from the context.

CSF-1 apparently occurs in numerous forms all of which are included in the embodiments of the present invention. Human CSF-1 cDNA clones coding for CSF-1 pre-pro-polypeptides of three different lengths (256 amino acids; 554 amino acids; and 438 amino acids) have been isolated from cells expressing the single CSF-1 gene (see commonly owned U.S. Patent No. 4,847,201 issued July 11, 1989 and U.S. Patent No. 4,868,119, issued September 19, 1989; Wong, G.G., et al., Science (1987) 235: 1504, Kawasaki, et al., Science (1985) 230:291; Ladner et al., Embo J (1987) 6:2693; Cerretti, D.P. et al., Molecular Immunol (1988) 25:761. The CSF-1 proteins useful in the therapies disclosed herein may also be processed by proteolysis including, for example, in the long form, at the Lys residue at 238, the Arg residue at 249, and the Arg residue at 411. It is believed that CSF-1 may occur in nature in one or more C-terminally deleted forms. In addition, CSF-1 proteins lacking the first two or four amino acids have been isolated in active form from the supernatant of the human cell line AGR-ON (equivalent to CEM-ON; ATCC No. CRL-8199; Takahashi, M., et al., Biochem Biophys Res Comm (1988) 152:1401 and U.S. Patent No. 4,675,291 issued June 23, 1987). CSF-1 protein comprising monomers ending at amino acid 145 are reported to have in vitro biological activity (European Patent (EP) Publication No. 261,592 published March 30, 1988). Some biological activity is reported for a dimeric CSF-1 protein composed of monomers ending at amino acid 132 (EP 328,061 published August 16, 1989). The monomeric CSF-1 polypeptide (whether clipped at the C-terminus or not) may also refold to form multimers, most frequently dimers.

Native human urinary CSF-1 has been isolated as a highly glycosylated dimer of 45-90 kD, depending on the source, method of measurement and identity of the reporter. The recombinantly produced unglycosylated CSF-1 reported by Wong, et al., (supra) appears to have a monomeric molecular weight of approximately 21 kD. On the other hand, the molecular weight calculated on the basis of the amino acid sequence deduced for the "short" 224 amino acid form of CSF (SCSF) by Kawasaki et al., (supra) (see also U.S. Patent No. 4,847,201 (supra) and commonly owned PCT Publication No. WO86/04607 published August 14, 1986) is on the order of 26 kD, while that of the "long" 522 amino acid form (LCSF) is calculated to be on the order of 55 kD (Wong, et al., (supra); Ladner et al., (supra); commonly owned EP 272,779, published June 29, 1988; and U.S. Patent No. 4,868,119 corresponding to PCT Publication No. WO87/06954, published November 19, 1987). When deleted constructs of these genes are expressed in E. coli (where glycosylation does not occur), they, of course, give rise to proteins of considerably lower molecular weight.

Other forms of CSF-1 proteins useful in the present invention include the polymer conjugated CSF-1 described in commonly owned U.S. Patent No. 4,847,325. The transmembrane region deletion mutants disclosed in EP 249,477 published December 16, 1987 and the glycosylation site deletion mutants disclosed in EP 272,779, supra , are also considered to be useful in the presently disclosed therapies.

Methods for the production of these various forms of CSF-1 from various sources are reported in U.S. Patent No. 4,879,227 issued November 7, 1989; WO 86/04587 published August 14, 1986; WO 89/10407 published November 2, 1989; WO 88/08003; supra and EP 276,551 published August 3, 1988.

It is, of course, well known that bacterially produced mature proteins which are immediately preceded by an ATG start codon may or may not include the N-terminal methionine, and it is shown in EP 272,779 (supra) that deletion of residues 1 and 2 (both glutamic acid) or residues 1-3 (Glu-Glu-Val) aids in this manner. Deletions are noted by a ▼ followed by the number of amino acids deleted from the N-terminal sequence, or by the number of amino acids remaining when residues are deleted from the C-terminal sequence. Thus, the N-terminal deletions referred to above having the first 2 and the first 3 residues deleted are designated N▼2 and N▼3, respectively. C-terminal truncations of CSF-1 resulting in proteins of 150, 158, 190 and 221 amino acids in length for example are referred to as C▼150, C▼158, C▼190 and C▼221, respectively. A 221 amino acid CSF-1 molecule derived from LCSF having an N-terminal deletion of 3 amino acids is denoted by LCSF/N▼3 C▼221, for example. Amino acid substitutions are designated by reference to the position of the amino acid which is replaced. For example, substitution of the cysteine residue at position 157 in Figure 4 of Ladner et al., (supra) by serine is refened to as CSF-1 Ser₁₅₇.

In summary, in addition to the N-terminal and C-terminal deletions and aggregations, individual amino acid residues in the chain may be modified by oxidation, reduction, deletion or other derivatization, and these proteins may also be cleaved and/or polymerized to obtain dimeric products that retain activity. Such alterations which do not destroy activity do not remove the protein sequence from the definition, and an specifically included as substantial equivalents. CSF-1 derived from other species may fit the definition of a protein having activity of "human CSF-1" by virtue of its display of the requisite pattern of activity as set forth above with regard to human substrate.

As is the case for all proteins, the precise chemical structure depends on a number of factors. As ionizable amino and carboxyl groups are present in the molecule, a particular protein may be obtained as an acidic or basic salt, or in neutral form. All such preparations which retain their activity when placed in suitable environmental conditions are included in the definition. Further, the primary amino acid sequence may be augmented by derivatization using sugar moieties (glycosylation) or by other supplementary molecules such as lipids, phosphate, acetyl groups and the like, more commonly by conjugation with saccharides, polyethylene glycols (PEGs), and polyoxyethylene glycols (POGs) as shown in U.S. Patent No. 4,847,325. Certain aspects of such augmentation are accomplished through post-translational processing systems of the producing host; other such modifications may be introduced in vitro. In any event, such modifications are included in the definition so long as the activity of the dimeric protein, as defined above, is not destroyed. It is expected, of course, that such modifications may quantitatively or qualitatively affect the activity, either by enhancing or diminishing the activity of the protein in the various assays.

Further, individual amino acid residues in the chain may be modified by oxidation, reduction, or other derivatization, and the protein may be cleaved to obtain fragments which retain activity. Such alterations which do not destroy activity do not remove the protein sequence from the definition.

Modifications to the primary structure itself by deletion, addition, or alteration of the amino acids incorporated into the sequence during translation can be made without destroying the activity of the protein. Such substitutions or other alterations result in proteins having an amino acid sequence which falls within the definition of proteins "having an amino acid sequence substantially equivalent to that of CSF-1." Indeed, human- and murine-derived CSF-1 proteins have nonidentical but similar primary amino acid sequences which display a high homology.

The use according to the invention may stimulate monocyte-precursor/macrophage cell production from progenitor marrow cells, thus enhancing the effectiveness of the immune system, and of stimulating such functions of these differentiated cells as the secretion of lymphokines in the mature macrophages:

Opportunistic infections can be contracted as a result of immunosuppression. For example, AIDS related opportunistic infections are described in Mills et al., 1990, Scientific American 263:51-57. Mills et al. show that common opportunistic infections are caused by the following agents: Cytomegalovirus, Pneumocystis carinii, Candida albicans, Varicella-Zoster virus, Epstein-Barr virus, Toxoplasma gondii, Mycobacterium avium, Cryptococcus neoformans, etc. The authors also describe various agents that are used

Additionally, it is envisioned that the invention may be used to treat Cryptococcal infections, such as cryptococcal meningitis. Cryptococcal meningitis is the most common form of fungal meningitis in the United States and the third most important agent of neurologic disease after Human Immunodeficiency Virus (HIV) and Toxoplasma gondii in AIDS patients. Cryptococcosis will develop at some point during their illness in 5 to 7% of patients with AIDS in the U.S. while disseminated cryptococcosis may develop in up to a third of African AIDS patients.

The therapeutic treatment for these opportunistic infections can be similar to that described below for other fungal diseases.

In addition, subjects could be supplied enhanced amounts of previously differentiated macrophages to supplement those of the indigenous system, which macrophages are produced by in vitro culture of bone marrow or from blood monocytes, or other suitable preparations followed in accordance with the invention. These preparations include those of the patient's own blood monocytes or bone marrow derived cells, which can be so cultured and returned for local or systemic therapy.

The fungal infections typically treated by the invention arise in immunosuppressed patients (for example, patients that have bone marrow transplants, AIDs, etc.), but can also occur in non-immunosuppressed individuals. Fungi from the following genuses that can be treated: Candida, Aspergillus, Cryptococcus, Histoplasma, Blastomyces, Coccidioides, Paracoccidioides, Mucor, Rhodotorula, Sporothrix, Dermatophytosis, Pseudallescheria, Prototheca, Rhinosporidium, or fungi that cause mycetoma or chromomycosis, etc. (see Principles and Practice of Infectious Diseases, 3rd Edition, Mandell et al., Churchell Livingtone [1990] pages 1942-2016).

Preferably, the CSF-1 is parenterally administered until the fungal infection is cleared as determined by negative culture results. The CSF-1 can be administered subcutaneously, by continuous infusion, by bolus injection, or by constant infusion. By "continuous infusion" is meant at lean 24 hours of dosing and by "constant infusion" is meant 24 hours or less of dosing. The CSF-1 is preferably administered by constant infusion lasting between 1 and 4 hours. The daily CSF-1 dose that is effective to treat fungal infections is preferably between 0.01 to 50 mg/M², more preferably between 0.05 to 10 mg/M², most preferably between 0.5 and 5 mg/M². Preferably, CSF-1 is administered for at least 14 days, more preferably for 21-59 days. Other dose schedules can also be used if the above treatment causes unwanted effects (however, minimal effects have been observed at doses of up to 30 mg/M²/day by i.v. bolus) or if more efficacious results can be shown.

The invention may be used to teat Candida and Aspergillus infections as described above and specifically as shown below. Cryptococcus infections such as cyptococcal meningitis can be treated similarly.

Examples of antifungal agents in accordance with the invention include: Amphotericin B, Fluconazole (Diflucan), Ketoconazole, Miconazole, and Intraconazole (see also Mandell et al. above at pages 361-370 or Mills et al. at pages 54-55). As an example of the clinical spectrum of one of the above antifungal agents, Amphotericin typically inhibits the following fungi and protozoa: Aspergillus fumigatus, Paracoccidioides brasiliensis, Coccidioides immitis, Cryptococcus neoformans, Histoplasma capsulatum, Mucor mucedo, Rhodotorula spp., Sporothrix shenckii, Blastomyces dermatitidis, Candida spp., Leishmania spp., Naegleria spp., and Acanthamoeba spp. The antifungal agents can be administered before, during, or after the CSF-1 is given. Doses of these agents are known to those of ordinary skill in the art and include daily dose ranges such as 0.4 to 0.6 mg/kg/day for Amphotericin B, 200 mg/day to 400 mg/day for Fluconazole, and 200 mg/day to 400 mg/day for Ketoconazole. These ranges are merely exemplary and are not to be construed as limiting in any way. Also, it is contemplated that CSF- 1 can be combined with other agents that are, or will be, effective at treating fungal infections. The antifungal agents can be conjugated to polymers to adjust their in vivo half-life and toxicity, see U.S. Serial No. 365,914.

The CSF-1 of the invention may be formulated in conventional ways standard in the art for the administration of protein substances. Administration by injection is one preferred route; and such formulations include solutions or suspensions, emulsions, or solid composition for reconstitution into injectables or gel formulations. Suitable excipients include, for example, Ringer's solution, Hank's solution, water, saline, glycerol, dextrose or mannitol solutions, and the like. While liquid solutions of CSF-1 may be used directly on or under wound dressings, reconstituted compositions are useful for salves, gel formulations, foams and the like for wound healing. Reconstituted gel formulations provide a controlled delivery system for CSF-1 at a wound site. Controlled release refers to drug release sufficient to maintain a therapeutic level over an extended period of time, such as up to 24 hours or more, preferably in the range of 24-72 hours. Increased contact time of growth factors may be necessary to achieve a significant increase in the rate of wound healing.

In addition, the CSF-1 of the invention may be preincubated with preparations of cells in order to stimulate appropriate responses, and either the entire preparation or the supernatant therefrom introduced into the subject. As shown hereinbelow, the materials produced in response to CSF-1 stimulation by various types of blood cells are effective against desired targets, and the properties of these blood cells themselves to attack invading organisms or neoplasms may be enhanced. The subject's own cells may be withdrawn and used in this way, or, for example, monocytes or lymphocytes from another compatible individual employed in the incubation.

As discussed previously and particularly with regard to the subject matter disclosed in U.S. Patent No. 4,847,201 (supra), the complete coding sequences for a number of human CSF-1 proteins are now available, and expression vectors applicable to a variety of host systems have been constructed and the coding sequence expressed. In addition to those expression systems provided in U.S. Patent No. 4,847,201, expression systems employing insect cells utilizing the control systems provided by baculovirus vectors have been described (Miller, D.W., et al., in Generic Engineering (1986) Setlow, J.K., et al., eds, Plenum Publishing, Vol. 8, pp. 277-279, U.S. Patent No. 4,745,051, issued May 17, 1988, and copending U.S. Serial No. 077,188, filed July 24, 1987. Insect cell-based expression can be in Spodoptera frugiperda. These systems are also successful in producing CSF-1. Mammalian expression has been accomplished in COS-7, CHO, mouse, and CV-1 cells, and also can be accomplished in COS-A2, hamster, and murine cells.

The variety of hosts available, along with expression vectors suitable for such hosts, permits a choice among post-translational processing systems, and of environmental factors providing conformational regulation of the protein thus produced. Thus, the availability of this information provides CSF-1 proteins in sufficient quantity for application in the various therapies discussed herein.

Among the vectors disclosed in the aforementioned patent publications, the plasmids pLCSF221A (which contains the gene encoding asp₅₉LCSF/N▼3C▼221) and pcCSF-17 (which contains the gene encoding SCSF) are preferred for procaryotic and eukaryotic expression, respectively, of human CSF-1. The plasmid pCSF221A (hereinafter E. coli (221)) transformed into E. coli strain DG116, was deposited with the ATCC on April 14, 1987 under the Accession No. 67390. The plasmid pcCSF-17 in E. coli MM294 was deposited with the ATCC on June 14, 1985 under the Accession No. 53149.

Also preferred are CSF-1 proteins which comprise the amino acid sequences containing the first 3-150 or 4-150 amino acids of SCSF and LCSF and the C-terminal deletions, such as LCSF/▼221.

The activity of CSF-1 was determined in the following examples using partially purified MIAPaCa CSF-1, murine L cell CSF-1, CV-1-produced recombinant material or E. coli-produced human CSF-1. CSF-1 was shown to enhance the production of interferon (IFN) and tumor necrosis factor (TNF) by induced human monocytes by up to 10 to 30-fold. CSF-1 also was demonstrated to stimulate macrophage antitumor toxicity in vitro, to inhibit tumor growth in vivo, to protect mice from lethal bacterial infection, to promote the repair of tissue damage in vivo, to inhibit the growth of cytomegalovirus in vivo, and to inhibit the growth of yeast in vivo.

### In Vivo Effect of CSF-1 on Candida Albicans

CSF-1 was administered to outbred CD1 mice (27-28g. females) as daily i.p. doses for 3 to 4 days before challenge with a lethal dose of C. albicans (1.5 x 10⁸ yeast cells/mouse, i.p.). This challenge dose resulted in a median survival time (MST) of 3.0 days in non-treated and saline treated mice. CSF-1 treated mice showed MST of 15 and 13 days at doses of 1.9 and 0.1 mg/kg, respectively. This 4-fold enhancement in survival time is significant at p=0.01 (Log Range Tests). To minimize any possible interference from endotoxin, highly purified CSF-1 was used which was virtually free of endotoxin (<0.05 ng/mg protein). It was also shown that the prophylactic effect was abrogated by heat-inactivation of CSF-1 test material. This protection was associated with increased numbers of peripheral blood circulating monocytes and neutrophils and a 2- to 3-fold increase in peritoneal macrophages. Therefore, CSF-1 enhanced host resistance against C. albicans infection and that this effect is probably mediated by activation of macrophages and neutrophils.

CSF-1 was also tested in an additional model in which C. albicans was delivered systemically (i.v.). CSF-1 at a dose of 1.9 mg/kg/day QD x 4 was administered either i.p. or i.v. 2 x 10⁵ cfu/mouse were injected i.v. 4 hours after the last dose of CSF-1. Either of these administrations resulted in a significant enhancement of survival when compared to saline injected control mice.

### Survival Time of Rats With Fungal Infections After CSF-1 Treatment

Using CSF-1 in conjunction with the antifungal agent fluconazole, we demonstrated that dailing bolus subcutaneous (SQ) injection of CSF-1 given therapeutically improved the median survival time of infected animals in a rat Candidiasis model from 5 days (in animals receiving fluconazole alone) to greater than 30 days (in animals receiving a combination of CSF-1 and fluconazole).

Male Fisher-344 rats weighing 200-250 grams were obtained from Charles River Laboratories. Rats were housed at 5 per cage and received water and laboratory chow *ad libitum*. Serological testing for adventitious viruses and mycoplasma is routinely negative in these animals.

*Candida albicans* strain ATCC No.18804 from the American Type Culture Collection (Bethesda, MD) was grown overnight at 37°C in trypticase soy broth and ten centrifuged. The blastospores were resuspended and aliquoted in 95% fetal bovine serum and 5% dimethyl sulfoxide and frozen at -80°C. On the day of each experiment two aliquots were pooled, and then diluted in phosphate buffered saline (PBS) to 4 x 10⁶ blastospores per mL for injection. Each experimental inoculum was plated on Yeast Extract Peptone agar to confirm the viable organism count.

Fisher-344 rats were infected with 2 x 10⁶ *Candida albicans* blastospores (0.5 mls) by i.v. injection into a lateral tail vein. In the CSF-1 efficacy studies, a single 0.3 mg/Kg dose of fluconazole (Diflucan, Pfizer, New York, NY) was administered subcutaneous (SQ) 2 hours later. Immediately following the fluconazole treatment, a 0.5 mL SQ dose of CSF-1 was given at a different site. CSF-1 treatment was continued for an additional 9 days for a total of 10 doses. Animals were assessed daily for mortality for 30 days.

The dose of *Candida albicans* needed to produce 100% mortality in untreated F-344 rats was determined to be 2 x 10⁶ blastospores (Table 17). This dose resulted in mortalities in 3 to 6 days, with a median survival time of 4 days.

Table 18 presents the results of four experiments in which fluconazole was combined with the therapeutic SQ administration of CSF-1 after *Candida albicans* infection. Doses of CSF-1 at 0.3 and 1.0 mg/Kg/day appear to be the most efficacious, resulting in a median survival time of >30 days, at least 6 times the median survival time of control animals (5 days).

**Table 17**

| Mortality Table of Fisher 344 Rats Infected with *Canadida Albicans* | | | | |
|---|---|---|---|---|
| No. of C. *albicans* Blastospores Injected (x 10⁻⁵) | No. of Animals Tested | Percent Overall Mortality | Median Survival Time (Days) | Mean Survival Time of Lethally Infected Animals (Days) |
| 1.25 | 5 | 0 | >30 | |
| 2.5 | 5 | 20 | >30 | 5.0 |
| 5 | 16 | 38 | >30 | 5.0 |
| 10 | 16 | 88 | 4 | 4.4 |
| 20 | 50 | 100 | 4 | 4.4 |
| 40 | 5 | 100 | 4 | 4.4 |
| 100 | 8 | 100 | 2 | 2.8 |
| 330 | 8 | 100 | 1 | 1.5 |
| 1000 | 4 | 100 | 1 | 1.0 |

**Table 18**

| Therapeutic Use of CSF-1 with Fluconazole in *Candida Albicans* Infected Rats | | | | | | | |
|---|---|---|---|---|---|---|---|
| CSF-1 Dose with 0.3 mg/kg Fluconazole* | Experiment Number Survivors/Total | | | | Survival Totals from 4 Experiments | Median Survival Time (Days) | % Survival >30 days |
| | #1 | #2 | #3 | #4 | | | |
| CSF-1 | | | | | | | |
| 0.0 mg/kg | 1/5 | 4/10 | 1/8 | 4/10 | 10/33 | 5 | 30% |
| 0.03 mg/kg | ND | ND | 1/5 | ND | 1/5 | 5 | 20% |
| 0.1 mg/kg | 3/5 | ND | 2/5 | 3/5 | 8/15 | >30 | 53% |
| 0.3 mg/kg | 4/5 | 5/5 | 3/5 | 3/5 | 15/20 | >30 | 75% |
| 1.0 mg/kg | 4/5 | 5/5 | 4/5 | 4/5 | 17/20 | >30 | 85% |
| 3.0 mg/kg | ND | 5/5 | 1/5 | 3/5 | 9/15 | >30 | 80% |
| 10.0 mg/kg | ND | ND | ND | 1/5 | 1/5 | 6 | 20% |
| Control-PBS (No fluconazole) | 0/10 | 0/10 | 0/5 | 0/6 | 0/31 | 4 | 0% |

| | | | | | | | |
|---|---|---|---|---|---|---|---|
| * CSF-1 therapy of a SQ infection daily for 10 days began on Day 0.2 hours post-infection. All test animals receive a single dose of fluconazole, 0.3mg/kg, 2 hours post-infection. | | | | | | | |
| ND = Not done. | | | | | | | |

### Resolution of Fungal Infections Using CSF-1

Eighteen patients with invasive fungal infection which was refractory to standard therapy were treated with CSF-1. Those patients who were referred to a bone marrow transplant (BMT) center were eligible for study, but patients with a malignancy in relapse were ineligible. No exclusions were made for diagnosis, age, graft versus host disease (GVHD) status or patient's overall condition. The clinical characteristics of the 18 patients are shown in Table 19.

The diagnosis of invasive fungal infection required demonstration of organisms in biopsy specimens, cultures' of closed body fluids (i.e., cerebral spinal fluid) or radiologic evidence for invasive disease in the presence of positive blood cultures. Demonstration of fungi solely in bronchoscopic lavage fluid, in gastrointestinal biopsy specimens, or in single blood cultures were considered insufficient proof of invasive disease. The infecting fungal organism, the organs involved, and diagnostic modalities used to follow the infections are shown in Table 19.

All patients were examined and had routine blood cultures taken daily, as well as having blood chemistries and complete blood cell counts performed daily. Generally, bone marrow aspirates and biopsies were performed prior to therapy to rule out malignancy. The fungal infections were re-evaluated using the initial diagnostic procedures on a periodic basis and at autopsy.

The study was designed as a Phase I dose escalation trial in which patients were enrolled consecutively. All patients were maintained on the best available anti-fungal therapy at the maximally tolerated dose. CFS-1 (specific activity approximately 3-10 x 10⁷ U/mg) was given at a dose between 0.05 to 2.0 mg/M². CSF-1 was administered in 100 ml of normal saline with 0.25% albumin by central venous catheter as a single two-hour intravenous infusion daily for seven days. If after seven days there was no evidence for a clinical response, the dose was doubled and administered for an additional seven days. Three dose escalations were allowed in a single patient. If antifungal efficacy was noted, CSF-1 was continued until resolution of the fungal infection could be documented. Patients treated with 2 mg/M² were maintained at that dose.

CSF-1 was well tolerated at all dose levels. There were no specific symptoms or changes in blood chemistries that could be ascribed to it. There were no significant adverse effects on the severity of pre-existing GVHD during the CSF-1 infusion course.

Eleven patients had invasive candidiasis, four had aspergillosis, two were diagnosed with yeast sp., and one was diagnosed with Rhodatorula (Table 19). Three patients received CSF-1 prior to BMT for progressive fungal disease that had not responded to amphorericin B. One of these patients had under undergone two sinus cavity resections for aspergillosis. However, CT scan demonstrated a residual sinus mass, and aspiration cultures continued to demonstrate aspergillus. When cavitating multi-lobar pulmonary nodules developed and the patient became progressively azotemic on 1 mg/kg of amphotericin, CSF-1 was started and the amphotericin dose was reduced to 0.5 mg/kg. After 35-days of therapy, the CT scan showed clearing of the sinus cavities and cultures for aspergillus became negative. All but the two largest cavitary pulmonary nodules resolved. Both pulmonary nodules were subsequently resected and shown to be free of fungal elements. The patient remains off antifungal therapy awaiting an unrelated donor BMT. The second patient treated prior to BMT received two grams of amphotericin for Candida albicans (involving the liver and spleen) without a clinical or radiologic response. After 35 doses of CSF-1, marked radiologic evidence of improvement was noted, and the liver was rebiopsied with no evidence of residual fungal infection. The third patient had progressive hepatic candidiasis. She had clinical and radiologic resolution of the fungal infection after receiving 28 doses of CSF-1.

Thirteen of the eighteen patients showed resolution or clinical improvement in their fungal infections (Table 19). Several patients had complete resolution of their fungal infections and were discharged from the hospital. Two patients were well enough to receive a bone marrow transplant after their infections cleared. Of the remaining five patients, one patient was not evaluable, another patient may not have had an invasive fungal infection and expired due to pancreatitis, and the remaining three patients showed no response, but received CSF-1 treatment for 8 days or less.

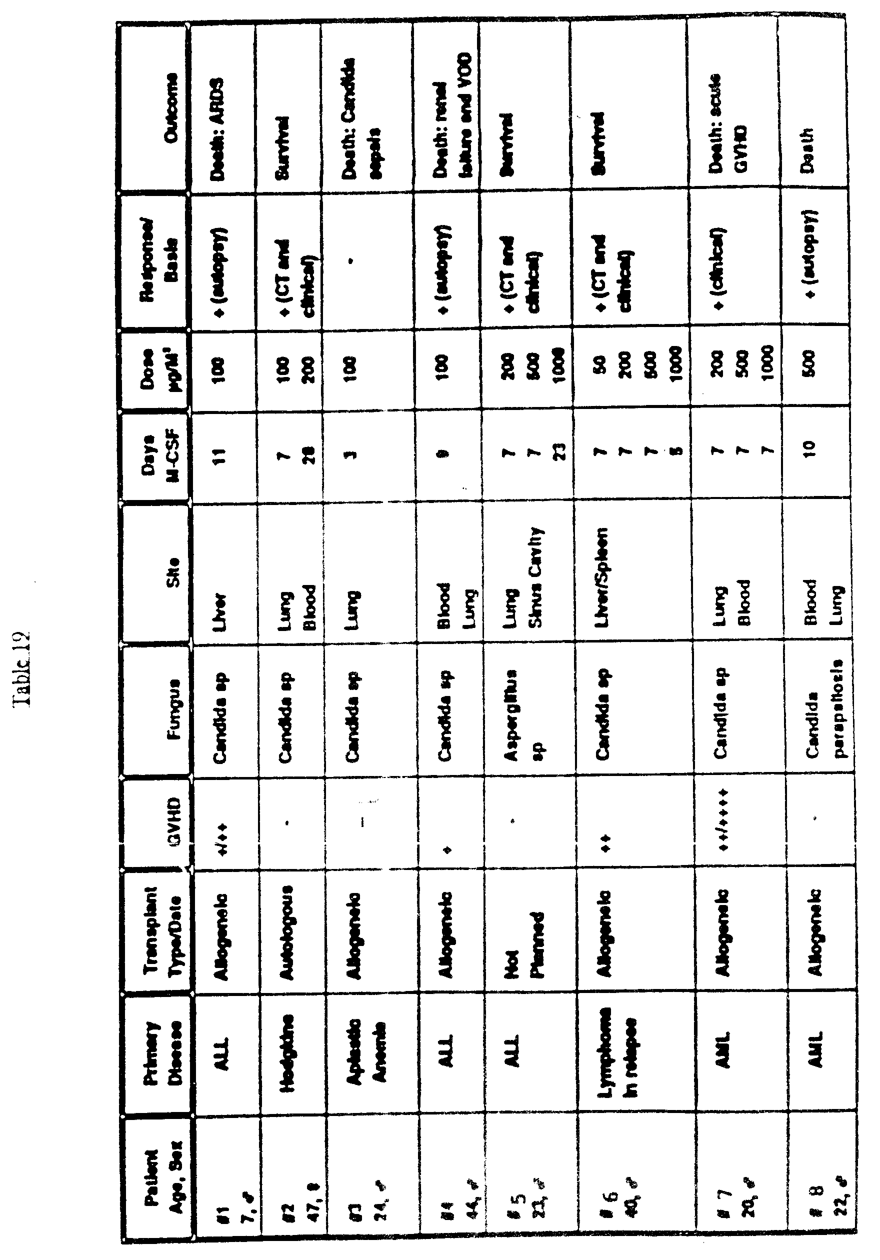

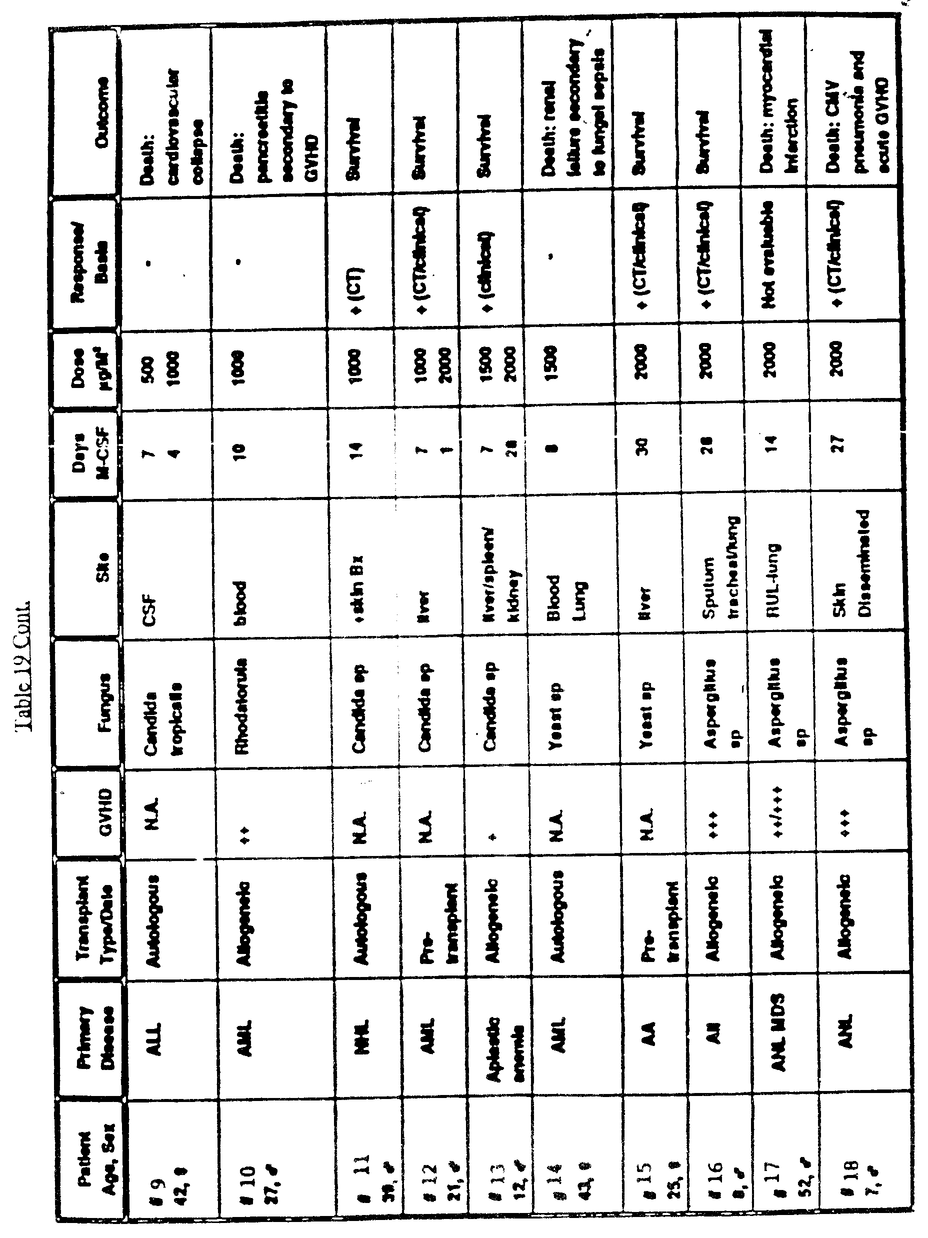

### Formulation and Dosage of CSF-1

The recombinantly produced human CSF-1 may be formulated for administration using standard pharmaceutical procedures. Depending on ultimate indication, CSF-1 will be prepared in an injectable or a topical form, As described above, the CSF-1 may interact in beneficial ways with appropriate blood cells, and the compositions of the invention therefore include incubation mixtures of such cells with CSF-1, optionally in the presence of additional lymphokines or cytokines. Either the supernatant fractions of such incubation mixtures, or the entire mixture containing the cells as well, may be used. Staggered timing may be preferred for some combinations, such as CSF-1 followed one to two days later by gamma interferon.

The CSF-1 described herein is generally administered therapeutically in amounts of between 0.01-10 mg/kg per day, whether single bolus administration or fractionated over 24 hr.

## Claims

1. The use of synergistically effective amounts of human colony stimulating factor 1 (CSF-1) and an anti-fungal agent, selected from amphotericin B, fluconazole, ketoconazole, miconazole or intraconazole for the manufacture of a medicament for the treatment of fungal infection.

2. The use as claimed in claim 1, wherein the medicament is for the treatment of fungal infection in immunosuppressed individuals.

3. The use as claimed in claim 2, wherein the immunosuppression of the individuals is due to infection including AIDS, to chemical agents including agents given for cancer chemotherapy or bone marrow transplant, to burn trauma, to other major trauma, or to irradiation.

4. The use as claimed in any one of claims 1 to 3, wherein the fungal infection is caused by at least one fungus selected from *Candida*, *Aspergillus*, *Cryptococcus*, *Histoplasma*, *Coccidioides*, *Paracoccidioides*, *Mucor*, *Rhodotorula*, *Sporothrix* or *Blastomyces* species.

5. The use as claimed in any one of claims 1 to 4, wherein the CSF-1 is administerable at a daily dose in the range 0.01 to 50 mg/M², preferably 0.05 to 10 mg/M², more preferably 0.5 to 5 mg/M², even more preferably 0.05 to 2.0 mg/M².

6. The us as claimed in any one of claims 1 to 4, wherein the CSF-1 is administerable at a daily dose in the range 0.05 to 2.0 mg/M².

7. The use as claimed in any one of claims 1 to 6, wherein the CSF-1 is administerable for at least 14 days.

## Patentansprüche

1. Verwendung von synergistisch wirksamen Mengen von menschlichem koloniestimulierendem Faktor 1 (CSF-1) und einem Antipilzmittel, ausgewählt aus Amphotericin B, Fluconazol, Ketoconazol, Miconazol oder Intraconazol, für die Herstellung eines Medikaments zur Behandlung einer Pilzinfektion.

2. Verwendung nach Anspruch 1, wobei das Medikament zur Behandlung einer Pilzinfektion in immunsupprimierten Personen dient.

3. Verwendung nach Anspruch 2, wobei die Immunsuppression der Personen durch eine Infektion, einschließlich AIDS, durch chemische Agenzien, einschließlich Agenzien für die Krebs-Chemotherapie oder Knochenmarkstransplantation, durch Brandwunden, andere größere Wunden oder durch Bestrahlung verursacht ist.

4. Verwendung nach einem der Ansprüche 1 bis 3, wobei die Pilzinfektion durch mindestens einen Pilz, ausgewählt aus den Arten Candida, Aspergillus, Cryptococcus, Histoplasma, Coccidioides, Paracoccidioides, Mucor, Rhodotorula, Sporothrix oder Blastomyces verursacht ist.

5. Verwendung nach einem der Ansprüche 1 bis 4, wobei das CSF-1 in einer täglichen Dosis im Bereich von 0,01 bis 50 mg/M², vorzugsweise 0,05 bis 10 mg/M², stärker bevorzugt 0,5 bis 5 mg/M², noch mehr bevorzugt 0,05 bis 2,0 mg/M² verabreichbar ist.

6. Verwendung nach einem der Ansprüche 1 bis 4, wobei das CSF-1 in einer täglichen Dosis im Bereich von 0,05 bis 2,0 mg/M² verabreichbar ist.

7. Verwendung nach einem der Ansprüche 1 bis 6, wobei das CSF-1 für mindestens 14 Tage verabreichbar ist.

## Revendications

1. Utilisation de quantités efficaces de manière synergique de facteur humain de stimulation de colonies 1 (CSF-1) et d'un agent antifongique, choisi parmi l'amphotéricine B, le fluconazole, le kétoconazole, le miconazole ou l'intraconazole pour la fabrication d'un médicament pour le traitement d'une infection fongique.

2. Utilisation selon la revendication 1, dans laquelle le médicament est destiné au traitement d'une infection fongique chez des individus immuno-déprimés.

3. Utilisation selon la revendication 2, dans laquelle l'imunodépression des individus est due à une infection incluant le SIDA, à des agents chimiques incluant des agents donnés lors de chimiothérapie cancéreuse ou de transplantation de moelle osseuse, à des traumatismes liés à des brûlures, à d'autres traumatismes majeurs, ou à une irradiation.

4. Utilisation selon l'une quelconque des revendications 1 à 3, dans laquelle l'infection fongique est causée par au moins un champignon choisi parmi les espèces *Candida*, *Aspergillus*, *Cryptococcus*, *Histoplasma*, *Coccidioldes*, *Paracoccidioides*, *Mucor*, *Rhodotorula*, *Sporothrix*, ou *Blastomyces*.

5. Utilisation selon l'une quelconque des revendications 1 à 4, dans laquelle le CSF-1 peut être administré à une dose quotidienne allant de 0,01 à 50 mg/m², de préférence 0,05 à 10 mg/m², de préférence encore 0,05 à 5 mg/m², et de préférence encore 0,05 à 2 mg/m².

6. Utilisation selon l'une quelconque des revendications 1 à 4, dans laquelle le CSF-1 peut être administré à une dose quotidienne allant de 0,05 à 2,0 mg/m².

7. Utilisation selon l'une quelconque des revendications 1 à 6, dans laquelle le CSF-1 peut être administré pendant au moins 14 jours.
